(19) Europäisches Patentamt
European Patent Office
Office européen des brevets

(11) Veröffentlichungsnummer: **0 024 588**
**B1**

(12) **EUROPÄISCHE PATENTSCHRIFT**

(45) Veröffentlichungstag der Patentschrift: **16.06.82**

(51) Int. Cl.³: **C 07 C 121/75,** C 07 C 120/00 // A01N53/00, A01N37/34

(21) Anmeldenummer: **80104626.9**

(22) Anmeldetag: **06.08.80**

(54) **Verfahren zur Herstellung von substituierten Cyclopropyl-carbonsäure-(alpha-cyano-3-phenoxy-benzyl)-estern.**

(30) Priorität: **18.08.79 DE 2933496**

(43) Veröffentlichungstag der Anmeldung: **11.03.81 Patentblatt 81/10**

(45) Bekanntmachung des Hinweises auf die Patenterteilung: **16.06.82 Patentblatt 82/24**

(84) Benannte Vertragsstaaten: **BE CH DE FR GB IT LI NL**

(56) Entgegenhaltungen:
**DE-A1-2 651 341**
**FR-A1-2 352 793**
**FR-A1-2 399 410**

(73) Patentinhaber: **BAYER AG, Zentralbereich Patente, Marken und Lizenzen, D-5090 Leverkusen 1, Bayerwerk (DE)**

(72) Erfinder: **Fuchs, Rainer, Dr., Roeberstrasse 8, D-5600 Wuppertal 1 (DE)**
Erfinder: **Maurer. Fritz, Dr., Roeberstrasse 8, D-5600 Wuppertal 1 (DE)**
Erfinder: **Priesnitz, Uwe, Dr., Severinstrasse 58, D-5650 Solingen 1 (DE)**
Erfinder: **Riebel, Hans-Jochem, Dr., In der Beek 92, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 024 588 B1

ACTORUM AG

# Verfahren zur Herstellung von substituierten Cyclopropyl-carbonsäure-(α-cyano-3-phenoxy-benzyl)-estern

Die Erfindung betrifft ein neues Verfahren zur Herstellung von bekannten substituierten Cyclopropylcarbonsäure-(α-cyano-3-phenoxy-benzyl)-estern.

Es ist bekannt, dass man (Cyclo-) Alkancarbonsäure-(α-cyano-3-phenoxy-4-fluor-benzyl)-ester, wie z.B. 3-(2,2-Dichlor-vinyl)- oder 3-(2-Chlor-2-(4-chlor-phenyl)-vinyl-2,2-dimethyl-cyclopropan-1-carbonsäure-(α-cyano-3-phenoxy-4-fluor-benzyl)-ester oder α-Isopropyl-α-(4-chlor-phenyl)-essigsäure-(α-cyano-3-phenoxy-4-fluorbenzyl)-ester, erhält, wenn man die entsprechenden Säurechloride, wie z.B. 3-(2,2-Dichlor-vinyl)- oder 3-(2-Chlor-2-(4-chlor-phenyl)-vinyl-2,2-dimethyl-cyclo-propan-1-carbonsäurechlorid oder α-Isopropyl-α-(4-chlor-phenyl)-essigsäurechlorid mit α-Cyano-3-phenoxy-4-fluor-benzylalkohol umsetzt (vergleiche DE-OS 2709264 und 2730515).

Ausbeute und Qualität der Produkte sind bei diesem Herstellungsverfahren jedoch unbefriedigend. Die Ursache hierfür dürfte hauptsächlich darin bestehen, dass der als Ausgangsverbindung eingesetzte α-Cyano-3-phenoxy-4-fluor-benzylalkohol sehr leicht in die Ausgangskomponenten, 3-Phenoxy-4-fluor-benzaldehyd und Cyanwasserstoff, zerfällt und praktisch nicht in reiner Form hergestellt werden kann.

Weiter ist bekannt, dass man substituierte Cyclopropancarbonsäure-α-cyano-benzylester erhält, wenn man substituierte Cyclopropancarbonsäurehalogenide mit substituierten Benzaldehyden in Gegenwart wässriger Lösungen von Natrium- oder Kalium-cyanid umsetzt (vergleiche DE-OS 2231312/US-PS 3835176).

Nach diesem Verfahren erhält man jedoch α-Cyano-benzylester ebenfalls nur in mässigen Ausbeuten.

Ferner ist bekannt, dass bei der Umsetzung von Säurechloriden mit substituierten Benzaldehyden und Alkalicyaniden die Ausbeuten an α-Cyano-benzylestern verbessert und die Reaktionszeiten stark verkürzt werden können, wenn die Umsetzungen in mehrphasigen Systemen aus wenig Wasser, gegebenenfalls festem Alkalicyanid und aprotischen Lösungsmitteln, gegebenenfalls unter Verwendung von Phasentransferkatalysatoren durchgeführt werden (vergleiche DE-OS 2708590/US-PS 4110360, 4110363 und 4123451).

Ferner ist aus DE-OS 2708590 bekannt, dass α-Phenylalkancarbonsäureester durch Umsetzung der entsprechenden Säurechloride, Alkalicyanid und Benzaldehyde im zweiphasigen Lösungsmittelsystem erhalten werden können, wenn spezielle Reaktionsbedingungen wie ein bestimmtes Verhältnis der Menge Wasser zu Cyanid angewendet werden. Die als Pestizide besonders interessanten Alkenylcyclopropancarbonsäure-α-cyanophenoxybenzylester können gemäss DE-OS 2708590 auf diese Weise nur bei Verwendung von Phasentransferkatalysatoren bei kurzer Reaktionszeit in hohen Ausbeuten hergestellt werden.

Es wurde nun gefunden, dass man substituierte Cyclopropylcarbonsäure-(α-cyano-3-phenoxy-benzyl)-ester der Formel I

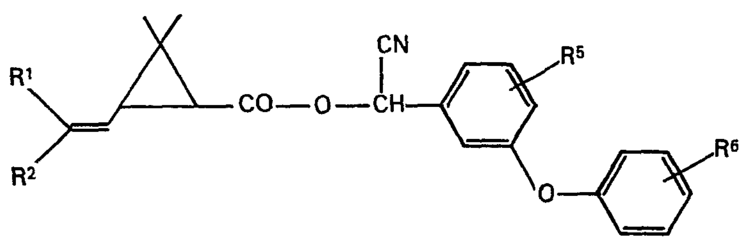

(I)

in welcher

$R^1$ und $R^2$ gleichermassen für Fluor, Chlor oder Brom stehen oder

$R^1$ für Fluor, Chlor oder Brom steht und

$R^2$ für Phenyl, 4-Fluor-phenyl oder 4-Chlorphenyl steht und

$R^5$ und $R^6$ für Wasserstoff oder Halogen stehen,

in hohen Ausbeuten erhält, indem man zu einer Mischung aus Cyclopropylcarbonsäurechloriden der Formel II

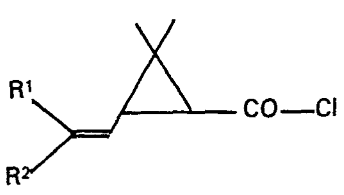

(II)

in welcher

$R^1$ und $R^2$ die oben angegebene Bedeutung haben, und 3-Phenoxy-benzaldehyden der Formel III

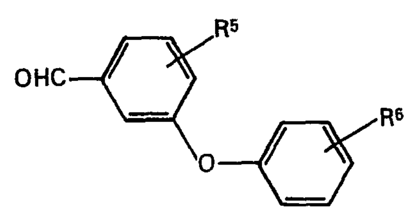

(III)

in welcher

$R^5$ und $R^6$ die oben angegebene Bedeutung haben, in einem mit Wasser praktisch nicht mischbaren Lösungsmittel aus der Reihe der Kohlenwasserstoffe eine wässrige Lösung oder Suspension von wenigstens der äquimolaren Menge

eines wasserlöslichen Cyanids bei Temperaturen zwischen 0 und 80°C unter heftigem Rühren zusetzt.

Vor allem in Anbetracht des aus der DE-OS 2708590 dargelegten Standes der Technik ist es als überraschend anzusehen, dass nach dem erfindungsgemässen Verfahren substituierte Alkenylcyclopropancarbonsäure-α-cyanobenzylester ohne Verwendung von Phasentransferkatalysatoren bei kurzer Reaktionszeit in praktisch quantitativen Ausbeuten hergestellt werden können.

Verwendet man als Ausgangsstoffe beispielsweise 3-(2,2-Dibrom-vinyl)-2,2-dimethyl-cyclopropan-1-carbonsäurechlorid und 3-Phenoxy-4-fluor-benzaldehyd sowie eine wässrige Lösung von Natriumcyanid neben Hexan als organischem Lösungsmittel, so kann die Reaktion dieser Verbindungen durch folgendes Formelschema skizziert werden:

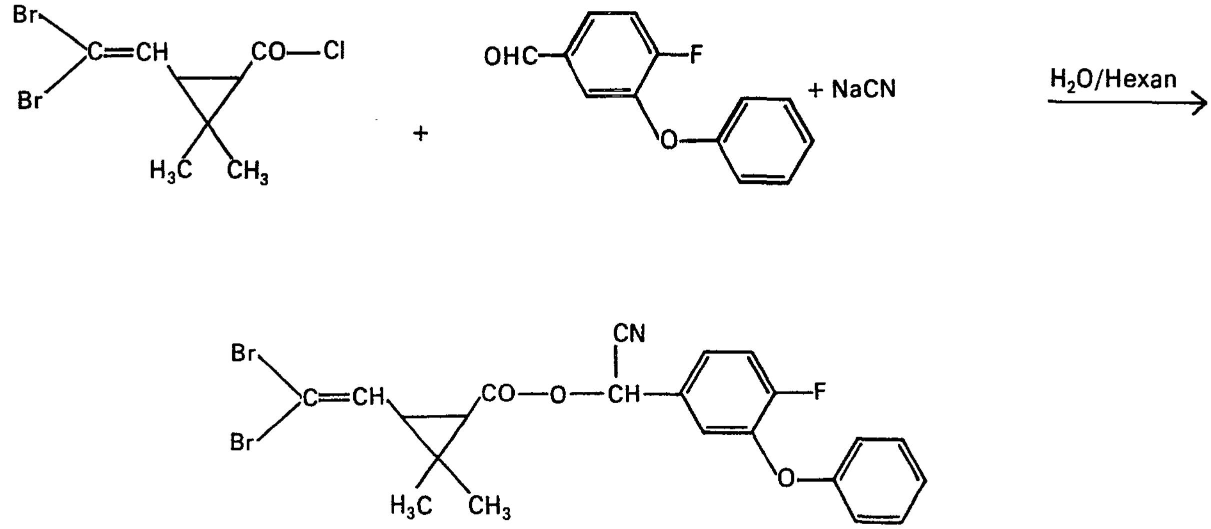


Bevorzugt stehen in den Benzaldehyden der Formel III
$R^5$ und $R^6$ für Wasserstoff oder Fluor.

Besonders bevorzugt stehen in den Benzaldehyden der Formel III
$R^5$ für Fluor und
$R^6$ für Wasserstoff.

Als Beispiele für die Ausgangsstoffe der Formel (II) seien genannt:
3-(2,2-Dichlor-vinyl)-, 3-(2,2-Dibrom-vinyl)-, 3-(2-Chlor-2-phenyl-vinyl)-, 3-(2-Chlor-2-(4-fluor-phenyl)-vinyl)- und 3-(2-Chlor-2-(4-chlor-phenyl)-vinyl)-2,2-dimethyl-cyclopropan-1-carbonsäurechlorid.

3-Phenoxy-4-fluor-benzaldehyd sei als Beispiel für die Ausgangsstoffe der Formel (III) genannt.

Die Ausgangsverbindungen der Formeln (II) und (III) sind bereits bekannt (vergleiche DE-OS 2709264 und 2730515, GB-PS 1413491 und 2000764 sowie US-PS 3835176 und 3962458).

In Wasser lösliche Cyanide, welche beim erfindungsgemässen Verfahren verwendet werden können, sind beispielsweise Natriumcyanid und Kaliumcyanid; Natriumcyanid wird bevorzugt verwendet.

Als mit Wasser nicht mischbare Lösungsmittel werden beim erfindungsgemässen Verfahren vorzugsweise geradkettige oder verzweigte Alkane oder Cycloalkane mit 5 bis 10 Kohlenstoffatomen, wie z.B. n-Pentan, n-Hexan, n-Heptan, n-Octan, n-Nonan, n-Decan, 2-Methylpentan, 3-Methylpentan, 2-Methylhexan, 2,2,4-Trimethylpentan, Cyclohexan, Methylcyclohexan, oder auch Methylbenzole, wie z.B. Toluol oder Xylole, sowie auch Gemische dieser Kohlenwasserstoffe eingesetzt.

Cyclohexan wird als Lösungsmittelkomponente besonders bevorzugt.

Die Reaktionstemperatur wird beim erfindungsgemässen Verfahren zwischen 0 und 80°C, vorzugsweise zwischen 10 und 50°C, insbesondere zwischen 15 und 35°C gehalten.

Das Verfahren wird gewöhnlich bei Normaldruck durchgeführt.

Auf 1 Mol Säurechlorid der Formel (II) werden im allgemeinen zwischen 0,8 und 1,1 Mol, vorzugsweise 0,9 bis 1,0 Mol 3-Phenoxy-benzaldehyd, 1 bis 2 Mol vorzugsweise 1,1 bis 1,4 Mol Cyanid, 50 bis 1500 ml, vorzugsweise 100 bis 1000 ml Wasser und 100 bis 3000 ml, vorzugsweise 200 bis 2000 ml des mit Wasser nicht mischbaren Lösungsmittels eingesetzt.

Die Reaktionszeit für einen Umsatz über 95% liegt im allgemeinen zwischen 1 und 5 Stunden.

Die Ausgangsverbindungen der Formeln (II) und (III) werden in dem mit Wasser nicht mischbaren Lösungsmittel gelöst und zu dieser Lösung wird unter starkem Rühren eine wässrige Lösung des Cyanids langsam zudosiert, wobei gegebenenfalls durch Aussenkühlung die Temperatur der Reaktionsmischung im oben angegebenen Bereich gehalten wird. Das komplette Gemisch wird noch eine oder mehrere Stunden gerührt. Zur Aufarbeitung wird gegebenenfalls mit weiterem mit Wasser nicht mischbarem Lösungsmittel verdünnt, die organische Phase abgetrennt, mit Wasser gewaschen, getrocknet und filtriert. Das

Filtrat wird durch Destillation unter vermindertem Druck vom Lösungsmittel befreit, wobei man das Produkt als öligen Rückstand erhält.

Die als bevorzugte Ausgangsverbindungen der Formel (II) genannten 3-Alkenyl-2,2-dimethyl-cyclopropan-1-carbonsäurechloride weisen asymmetrische Kohlenstoffatome auf und können, wie auch die entsprechenden Produkte der Formel (I) in einer entsprechenden Anzahl von stereoisomeren Formen auftreten.

Die in den Ausgangsverbindungen vorgegebene Konfiguration am Cyclopropangerüst bleibt beim erfindungsgemässen Verfahren praktisch vollständig erhalten.

Die nach dem erfindungsgemässen Verfahren herzustellenden (Cyclo-)Alkancarbonsäure-(α-cyano-3-phenoxy-benzyl)-ester können als Schädlingsbekämpfungsmittel verwendet werden (vergleiche DE-OS 2 709 264 und 2 730 515).

Beispiel

Zu einer Lösung von 45 mMol 3-Phenoxy-4-fluor-benzaldehyd und 50 mMol eines Säurechlorids der Formel (II) in Cyclohexan wird unter heftigem Rühren bei Eiskühlung eine Lösung von Natriumcyanid in Wasser tropfenweise gegeben. Das Gemisch wird drei Stunden bei 25°C gerührt. Anschliessend wird mit Cyclohexan verdünnt, die organische Phase abgetrennt, mit Wasser gewaschen, über Natriumsulfat getrocknet und filtriert. Vom Filtrat wird das Lösungsmittel sorgfältig abdestilliert.

Die variierten Mengen der eingesetzten Reaktionskomponenten und die Ausbeuten sind der nachstehenden Tabelle zu entnehmen.

Tabelle zum Beispiel (Mengenbilanz)

| Säurechlorid | NaCN (mMol) | Cyclohexan (ml) | Wasser (ml) | Ausbeute (% der Theorie) |
|---|---|---|---|---|
| 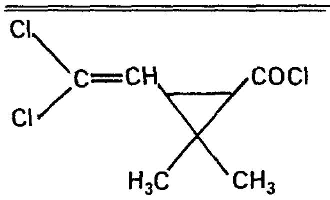 | 65 | 10 | 7 | 94,5 |
| 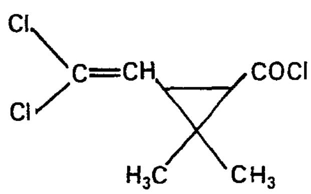 | 60 | 100 | 5 | 95 |
| 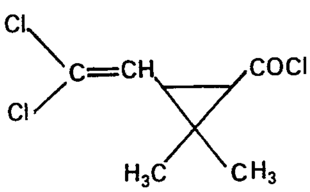 | 60 | 50 | 20 | 97 |
| 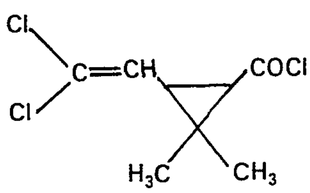 | 60 | 50 | 50 | 97 |
| 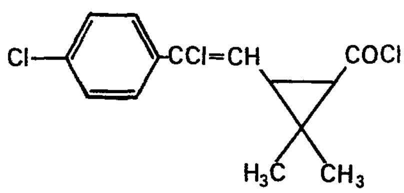 | 60 | 100 | 5 | 86 |
| 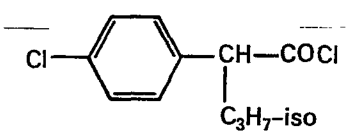 | 60 | 100 | 5 | 99 |

**Patentanspruch**

1. Verfahren zur Herstellung von substituierten Cyclopropancarbonsäure-(α-cyano-3-phenoxy-benzyl)-estern der Formel I

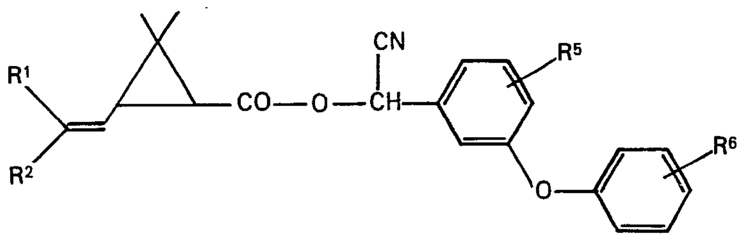

(I)

in welcher
$R^1$ und $R^2$ gleichermassen für Fluor, Chlor oder Brom stehen oder
$R^1$ für Fluor, Chlor oder Brom steht und
$R^2$ für Phenyl, 4-Fluor-phenyl oder 4-Chlorphenyl steht und
$R^5$ und $R^6$ für Wasserstoff oder Halogen stehen
dadurch gekennzeichnet, dass man zu einer Mischung aus Cyclopropylcarbonsäurechloriden der Formel II

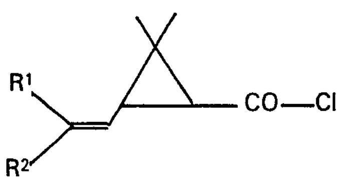

(II)

in welcher
$R^1$ und $R^2$ die oben angegebene Bedeutung haben,
und 3-Phenoxy-benzaldehyden der Formel III

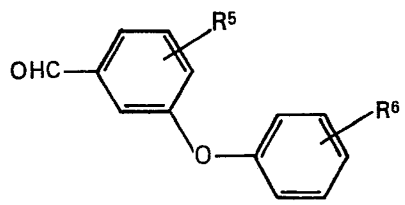

(III)

in welcher
$R^5$ und $R^6$ die oben angegebene Bedeutung haben, in einem mit Wasser praktisch nicht mischbaren Lösungsmittel aus der Reihe der Kohlenwasserstoffe eine wässrige Lösung oder Suspension von wenigstens der äquimolaren Menge eines wasserlöslichen Cyanids bei Temperaturen zwischen 0 und 80°C unter heftigem Rühren zusetzt.

**Claims**

1. Process for the preparation of substituted cyclopropanecarboxylic acid α-cyano-3-phenoxy-benzyl esters of the formula I

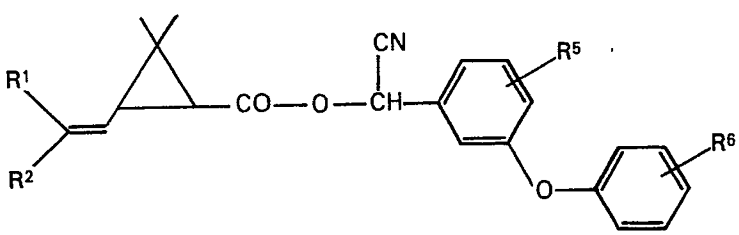

(I)

in which
$R^1$ und $R^2$ each represent fluorine, chlorine or bromine or
$R^1$ represents fluorine, chlorine or bromine and
$R^2$ represents phenyl, 4-fluoro-phenyl or 4-chlorphenyl and
$R^5$ and $R^6$ represent hydrogen or halogen
characterised in that an aqueous solution or suspension of at least an equimolar amount of a water-soluble cyanide is added to a mixture of cyclopropylcarboxylic acid chlorides of the formula II

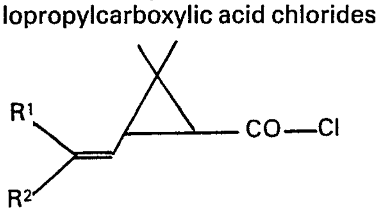

(II)

in which
$R^1$ and $R^2$ have the meaning given above, and 3-phenoxy-benzaldehydes of the formula III

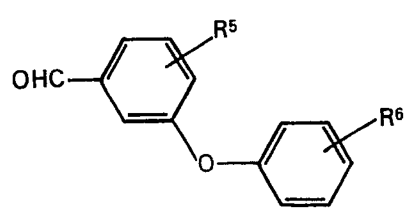

(III)

in which
$R^5$ and $R^6$ have the meaning given above, in a solvent from the hydrocarbon series which is virtually immiscible with water, at temperatures between 0 and 80°C with vigorous stirring.

**Revendication**

1. Procédé pour produire des cyclopropane carboxylates d'α-cyano 3-phénoxy benzyles substitués, esters répondant à la formule I:

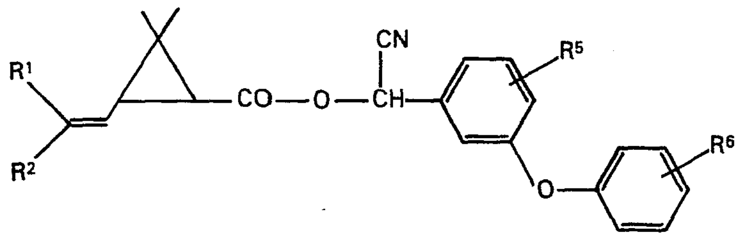

(I)

(dans laquelle:
$R^1$ et $R^2$ représentent de manière égale du fluor, du chlore ou du brome, ou bien
$R^1$ représente du fluor, du chlore ou du brome, et
$R^2$ représente un groupe phényle, 4-fluoro phényle ou 4-chloro phényle, et
$R^5$ et $R^6$ représentent de l'hydrogène ou un halogène),
procédé caractérisé en ce qu'à un mélange de chlorures d'acides cyclopropylcarboxyliques de formule II)

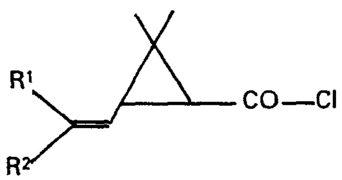

(II)

(dans laquelle:
$R^1$ et $R^2$ ont le sens indiqué ci-dessus),

et de 3-phénoxy benzaldéhydes de formule III:

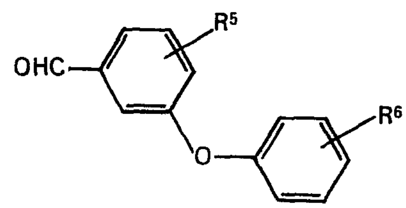

(III)

(dans laquelle:
$R^5$ et $R^6$ ont le sens indiqué ci-dessus), dans un solvant pratiquement non miscible à l'eau, de la série des hydrocarbures, on ajoute sous agitation puissante à des températures comprises entre 0 et 80°C une solution ou suspension aqueuse d'au moins la quantité équimolaire d'un cyanure hydrosoluble.